(19) Europäisches Patentamt European Patent Office Office européen des brevets

(11) **EP 2 819 528 B1**

(12) **EUROPEAN PATENT SPECIFICATION**

(45) Date of publication and mention of the grant of the patent:
**25.10.2017 Bulletin 2017/43**

(51) Int Cl.:
*A23J 3/10* (2006.01)   *A23J 3/08* (2006.01)
*A23L 33/19* (2016.01)   *A23L 5/00* (2016.01)

(21) Application number: **13710617.5**

(22) Date of filing: **28.02.2013**

(86) International application number:
**PCT/NL2013/050124**

(87) International publication number:
**WO 2013/129925 (06.09.2013 Gazette 2013/36)**

(54) **ENERGY-RICH LIQUID NUTRITIONAL COMPOSITION HAVING IMPROVED ORGANOLEPTIC PROPERTIES**

ENERGIEREICHE FLÜSSIGE ZUSAMMENSETZUNG MIT VERBESSERTEN ORGANOLEPTISCHEN MERKMALEN

COMPOSITION NUTRITIONNELLE LIQUIDE RICHE EN ENERGIE AYANT DES PROPRIETES ORGANOLEPTIQUES AMELIOREES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2012 PCT/NL2012/050121**

(43) Date of publication of application:
**07.01.2015 Bulletin 2015/02**

(73) Proprietor: **N.V. Nutricia**
**2712 HM Zoetermeer (NL)**

(72) Inventors:
• **DE KORT, Esther Jacqueline Petra**
**NL-3584 CT Utrecht (NL)**
• **BOTELHO DUTRA, Isabela**
**Sao Paulo / SP**
**CEP: 05435-020 (BR)**

(74) Representative: **Nederlandsch Octrooibureau**
**P.O. Box 29720**
**2502 LS The Hague (NL)**

(56) References cited:
**WO-A1-2009/072869   WO-A1-2010/140877**
**WO-A2-2004/054371   WO-A2-2009/101612**

**Description**

**FIELD OF THE INVENTION**

The present invention is in the field of enteral nutritional compositions. More in particular, the present invention relates to modulation of viscosity of liquid nutritional compositions. More in particular, viscosity of liquid nutritional compositions having a neutral pH, comprising high protein levels and micellar casein. More in particular, the invention addresses the problem of reducing viscosity, thereby facilitating the provision of energy-rich, liquid nutritional compositions suitable for enteral feeding and/or for special medical purposes.

**BACKGROUND OF THE INVENTION**

Preferably, medical dairy products are highly concentrated in nutrients, in particular in proteins and minerals, to meet the daily intake of nutrients in malnourished patients. These patients can be cachectic patients or persons suffering from end-stage AIDS, cancer or cancer treatment, severe pulmonary diseases like COPD (chronic obstructive pulmonary disease), tuberculosis and other infection diseases or persons that experienced severe surgery or trauma like burns. Furthermore, persons suffering from disorders in the throat or mouth such as oesophageal cancer or stomatitis and persons having problems with swallowing like dysphagic persons, require special liquid, low-volume nutrition. Also, persons just suffering from reduced appetite or loss of taste, will benefit from low-volume, preferably liquid, food. These patients can also be elderly persons, in particular frail elderly and elderly at risk of becoming frail. In this regard, although an elderly person's energy needs may be reduced, their ability to consume products may also be diminished. For example, they may have difficulty consuming a product due to, e.g., swallowing difficulties, or due to too large amount of product they need to consume to meet the daily intake of nutrients. Hence, compliance is not optimal, and often, the intake is suboptimal, leading to suboptimal nourishment, and in the end, to malnutrition.

The aforementioned groups of patients may be extremely sensitive to food consistency and to the organoleptic properties of the product such as viscosity, stickiness, mouth feel, taste, smell and colour. Also, patients such as cachectic patients, typically suffer from extreme weakness which often prevents them from sitting in a vertical position and from drinking food from a carton or even to suck it from a straw. These patients benefit well from liquid, low-volume enteral compositions with high nutrient content, in particular protein.

However, high amounts of protein and minerals increase the overall viscosity of the product during processing and storage. Low viscous liquid products, however, are mostly appreciated by patients, which makes it challenging to formulate such products.

Therefore, the problem underlying the present invention is to provide a liquid enteral composition for providing nutrition, either as a supplement, or as a complete nutrition, comprising a high protein content, in particular micellar casein as major protein source, in a small volume of liquid, and which supports nutrition and well-being in the different patient groups mentioned above, in particular to an elderly person or an ill patient. It is also an object of the invention to provide an improved manufacturing process, avoiding or diminishing any high viscosity-associated issues.

**PRIOR ART**

Prior art documents are available which are concerned with cheese-making processes wherein milk proteins, such as micellar casein or whey, are related to lactic acid (producing bacteria) or citric acid for acidifying and processing of compositions used for making cheeses. For instance, in WO200230210, a method of manufacturing cheese from milk is described wherein micellar casein, lactic acid and citric acid are mentioned. Both acids are mentioned as being equally well suited for the purpose of acidifying the composition. Such intermediates in cheese manufacture are not suited for the targeted patient group; also, viscosity is not an issue there.

WO 2004/054371 relates to a nutritional liquid composition which is chemically acidified with lactic acid for preventing the growth of pathogenic bacteria. The pH is below 3.5 and 6. It does not concern any viscosity issues associated with high-protein concentrations, as in fact the composition is directed to reconstituted infant formulae dealing with relatively low protein concentrations, i.e. about 1.3-1.5 g/100 ml. Acidification is strived for.

WO2010/140877 relates to liquid enteral nutritional compositions containing micellar casein and optionally caseinate, and in which the total amount of monovalent metal ions is less than 25 mg/g of protein. Organic acids may be selected to be included in the composition, and amongst all organic acids mentioned it is citric acid that is the favorite and used in the examples.

**SUMMARY OF THE INVENTION**

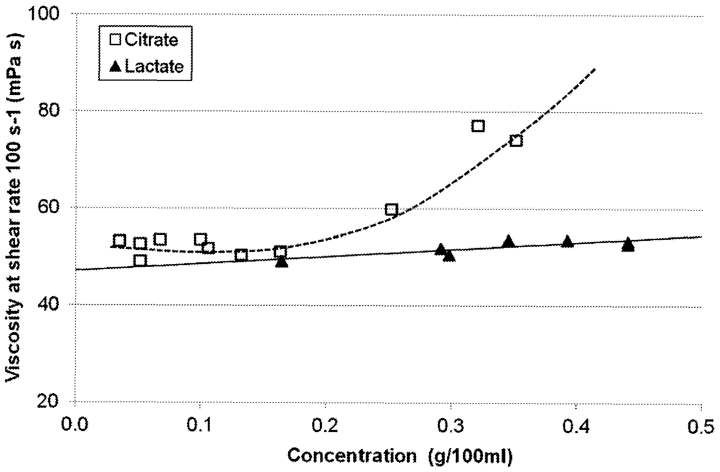

The present invention relates to a liquid nutritional composition with improved organoleptic properties compared to existing compositions. Said improved composition has a pH in a range of 6 to 8, comprises 6 to 20 g/100 ml protein, wherein this protein comprises at least 55 wt% micellar casein and further comprises lactic acid, in an amount between 0.05 and up to 1.5 g/100 ml, and is designed to meet the nutritional needs of persons, in particular persons in need thereof, such as elderly and patients with certain disease states. The composition, while being energy-rich with in particular a high protein content, has improved organoleptic properties, in particular lowered viscosity, to allow the composition to be better consumed orally or be administered by tube. Improving the organoleptic properties allows for increased compliance when consumption by patients of such compositions is involved. It was found that viscosity control could be achieved using lactic acid, while citric acid traditionally used for stabilizing such high-protein and/or high-energy compositions in the field had the opposite effect. The inventors' findings are explained in more detail in the examples attached. Reference is made to Figure 1.

## Liquid nutritional composition

Therefore, the present invention relates to a liquid nutritional composition having a pH in a range of 6 to 8, comprising 6 to 20 g/100 ml protein, said protein comprising micellar casein, wherein the amount of micellar casein is at least 55 wt% based on total protein content, and lactic acid, and wherein lactic acid is present in an amount between 0.05 and up to 1.5 g/100 ml.

Preferably, the amount of protein in said liquid nutritional composition lies between 7 and 20 g/100 ml, more preferably between 8 and 19 g/100 ml, even more preferably between 9 and 18 g/100 ml, most preferably between 9 and 16 g/100ml.

The amount of micellar casein in said liquid nutritional composition lies preferably between 55 and 95 wt%, more preferably between 60 and 90 wt%, more preferably between 65 and 85 wt% based on total protein content.

In a preferred embodiment, the liquid nutritional composition of the present invention further comprises caseinate, preferably less than 40 wt%, more preferably less than 35 wt%, based on total protein content. In one embodiment, caseinates are present in an amount ranging between 1 and 40 wt%, more preferably 2-35 %.

Preferably, the liquid nutritional composition of the invention comprises lactic acid in an amount between 0.05 and 1.0 g/100 ml, more preferably between 0.1 and 1.0 g/100ml, most preferably between 0.2 and 0.5 g/100ml of the total liquid composition. Inclusion of lactic acid in a liquid nutritional composition according to the invention instead of equivalent amounts of citric acid in the indicated range, was shown to reduce viscosity of compositions according to the invention. The increase in viscosity by citric acid was most pronounced at a concentration above 0.2 g/100ml, whereas when lactic acid was present in that concentration, viscosity did not show such a sharp increase, but in fact stabilized.

Alternatively, the amount of lactic acid is linked to the protein content of the product, since it is after all the proteins that otherwise contribute to the viscosity issue to a large extent. Preferably, the amount of lactic acid is up to 250mg/g protein. More preferably, the amount of lactic acid lies between 1 and 200 mg per gram protein of the total liquid composition, preferably between 2.5 and 100 mg/g protein, more preferably between 5 and 75 mg/g protein, most preferably between 10 and 75 mg lactic acid, per gram protein of the total liquid composition.

Alternatively, the liquid nutritional composition of the invention comprises preferably an amount of lactic acid up to 400 mg/g micellar casein of the total liquid composition. More preferably, the amount of lactic acid lies between 4 and 300 mg/g protein, more preferably between 10 and 200 mg/g micellar casein, most preferably between 20 and 100 mg lactic acid per g micellar casein of the total liquid composition.

In a preferred embodiment, the composition of the present invention further comprises citrate, preferably in an amount up to 1 g/100ml, preferably in an amount between 1 mg and 500 mg/100ml, more preferably in an amount between 5 mg and 400 mg/100ml, more preferably in an amount between 10 mg and 300 mg/100ml, most preferably in an amount of 15 mg and 100 mg/100ml of the total liquid composition. It is beneficial to include a certain amount of citrate in the liquid nutritional composition of the invention for prolonged heat-stability and shelf-life.

Preferably, the composition comprises a combination of citric acid and lactic acid. The combined amount thereof is preferably up to 2.5 g/100ml, more preferably this combined amount lies between 0.05 and 2 g/100ml, more preferably between 0.1 and 1.5 g/100ml, even more preferably between 0.25 and 1.0 g/100ml, most preferably between 0.3 and 0.75 g/100m1. Despite the significant amounts of citric acid present in these embodiments, it was found that lactic acid stabilized viscosity levels and largely made up for the viscosity effects that would have been observed when using citric acid alone.

In case lactic acid an citric are both present, the weight amount of lactic acid preferably exceeds the weight amount of citrate, preferably by a factor 1.1 to 20, more preferably by a factor 2 to 18, more preferably a factor 3 to 15 or most preferably 4 to 12. At such ratios, viscosity of the liquid nutritional composition is kept low, while other parameters, such as shelf-life and heat-stability influenced by the presence of citric acid, are kept at sufficient levels.

The energy density of the liquid nutritional composition of the present invention preferably lies between 1.2 and 3.5 kcal/ml, preferably between 1.4 and 3.0 kcal/ml, more preferably between 1.8 and 2.8 kcal/ml.

In a preferred embodiment of the present invention, the liquid nutritional composition further comprises at least one monovalent metal ion, in particular sodium, potassium or a mixture thereof. Preferably, the total amount of monovalent metal ions comprised by the liquid composition lies between 50 and 700mg/100ml, preferably between 100 and 600, more preferably between 125 and 500, most preferably between 125 and 400. Preferably, the monovalent metal ion is sodium, potassium or a mixture thereof.

Where the liquid nutritional composition comprises at least one monovalent metal ion, the total amount of monovalent metal ions comprised by the liquid composition preferably lies between 25 and 400mg/100 kcal, preferably between 30 and 300, more preferably between 40 and 350, most preferably between 50 and 300 mg/100 kcal.

In a further preferred embodiment, the liquid nutritional composition further comprises at least one divalent metal ion, preferably calcium and/or magnesium. The total amount of divalent metal ions is preferably present in an amount of between 10 and 600 mg/100ml, preferably between 50 and 550 mg/100 ml, more preferably between 100 and 500 mg/100ml

Alternatively, the total amount of divalent metal ions is preferably present in an amount of between 30 and 400 mg/100kcal, preferably between 50 and 350 mg/100 kcal, more preferably between 60 and 300 mg/100 kcal

The total amount of mono- and divalent metal ions in the liquid nutritional composition preferably lies between 60 and 1300 mg/100m1, more preferably this amount lies between 100 and 1200 mg/100ml, even more preferably between 125 and 1000 mg/100m1, most preferably between 225 and 900 mg/100ml.

Preferably, the liquid nutritional composition is obtained or obtainable by subjecting said composition to heat-treatment, such as sterilization (e.g. by ultra-high temperature treatment) or pasteurization.

The liquid nutritional composition of the invention has a viscosity which is preferably lower than 200 mPa.s, preferably lower than 150 mPa.s, more preferably lower than 100 mPa.s, most preferably lower than 80 mPa.s, as measured at a shear rate of 100 s$^{-1}$ at 20 °C using a rotational viscosity meter using a cone/plate geometry.

Also encompassed by the present invention is a powder, obtainable by drying the liquid enteral nutritional composition of the present invention.

Further encompassed by the present invention is the use of lactic acid in reducing viscosity of liquid nutritional compositions. said compositions having a pH in a range of 6 to 8 and comprising 6 to 20 g/100 ml protein, said protein comprising at least 55 wt% micellar casein, wherein the lactic acid is present in an amount between 0.05 and up to 1.5 g/100 ml. Further encompassed by the present invention is the liquid nutritional composition of the invention for use in treating a person of the age of 50 or more, a person that is in a disease state, a person that is recovering from a disease state, or a person that is malnourished.

Further encompassed by the present invention is a method of reducing viscosity of a liquid nutritional composition having a pH in a range of 6 to 8, comprising 6 to 20 g/100 ml protein of which at least 55 wt% is micellar casein, comprising the step of including lactic acid in said composition, wherein the lactic acid is present in an amount between 0.05 and up to 1.5 g/100 ml, and a liquid nutritional composition obtainable by said modulation process.

## DETAILED DESCRIPTION OF THE INVENTION

### Liquid nutritional composition

The inventors have now found that the viscosity of a micellar casein comprising liquid nutritional composition, preferably an aqueous composition, comprising 6 to 20 g/100 ml protein, and having a pH of about 6 to 8, can be modulated by adjustment of lactic acid levels. Since viscosity of such energy-rich, enteral compositions can be problematically high for certain patient groups, elderly and critically-ill, it is preferred that these two properties of liquid enteral nutritionial compositions are better understood to allow improved control thereof. Such control may aid in the provision of compositions having a sufficiently low viscosity to allow it to be consumed by persons that may have difficulty swallowing products.

Additionally or alternatively, it is preferred that viscosity of existing energy-rich, nutritional compositions can be reduced to allow inclusion of even higher nutritient levels, thereby obtaining a similar viscosity of the end product. Additionally, from a technological point of view it is preferred that viscosity of liquid nutritional compositions, especially for enteral use, can be better controlled to allow balancing out those parameters with nutritional densities, in particular micellar casein protein levels, for better design of liquid nutritional compositions for enteral use.

Preferably, the liquid nutritional composition according to the invention comprises at least 6, 7, 8, 9, 10, 11, 12, and at most 13, 14, 15, 16, 17, 18,19, 20 g protein per 100 ml of composition, such as 7 to 20 g/100 ml, preferably 8 to 19 g/100 ml, more preferably 9 to 18 g/100 ml, most preferably 9 to 16 g/100m1. The amount of micellar casein thereof is at least 55wt%, more preferably between 55 and 95 wt%, even more preferably between 60 and 90 wt%, most preferably between 65 and 85 wt, based on total protein content of the composition.

In a preferred embodiment, the liquid nutritional composition of the present invention further comprises caseinate, preferably at most 40 wt%, more preferably at most 35 wt%, based on total protein content. In one embodiment, caseinates are present in an amount ranging between 1 and 40 wt%, more preferably 2 - 35 %.

The pH of the aquous micellar casein composition should be between about 6 and 8. The pH is determined in the liquid nutritional composition of the invention and this can be done by routine methods, known to the skilled person, such as using a commercially available pH metering device. In further embodiments, the composition has a pH ranging between 6.1 and 7.8, preferably between 6.2 and 7.5, more preferably between 6.3 and 7.3.

The term "lactic acid" herein is interchangeable with the term "lactate" and is meant to include lactic acid as well as its salt form, *i.e.* lactate, such as sodium lactate, potassium lactate, magnesium lactate, calcium lactate or a combination thereof. Due to the intrinsic properties of lactate, this compound is present in the liquid composition of the invention with lactic acid in a salt-acid equilibrium at the indicated pH range. In the context of the invention, where amounts of lactic acid are mentioned, these refer to the amount of the lactic acid and/or lactate, without the accompanying metal salt ion. It is considered within the skilled persons ambit to correct for any cation contributions.

The term "citric acid" herein is interchangeable with the term "citrate" and is meant to include citric acid, as well as its salt form, i.e. citrate, such as magnesium citrate, calcium citrate, preferably potassium citrate, sodium citrate or a combination thereof. Due to the intrinsic properties of citrate, this compound is present in the liquid composition of the invention with citric acid in a salt-acid equilibrium at the indicated pH range. In the context of the invention, where amounts of citric acid are mentioned, these refer to the amount of the citric acid and/or citrate, without the accompanying metal salt ion.

Within the context of the present invention, "micellar casein", sometimes also referred to as "native micellar casein", refers to casein in the form of micelles, which is the native form of casein in milk. It is a high quality milk protein and naturally occurring in milk in a concentration of about 2.6 g/100 ml (Dairy Science and Technology, Walstra et al., CRC Press, 2006, table 1.1, page 4). It is concentrated by a process that does not, or does not substantially influence the native, micellar structure of the casein proteins. Casein micelles are commercially available with varying amounts of calcium, but in general have a high calcium-content in the order of about 25 g/kg protein. Micellar casein is commercially available in the form of, for example, micellar casein isolate or micellar protein concentrate.

In contrast, casein, as it is used in the context of this invention, refers to the curd form of casein, having lost its native micellar structure. It is bound to a metal, such as sodium, potassium, calcium and magnesium, and is commonly called caseinate. To avoid any confusion, in the remainder of the application the non-micellar casein will be referred to as 'caseinate' herein below.

According to one embodiment, the liquid nutritional composition comprises whey protein. Preferably, at a concentration of not more than 15 wt%, more preferably at most 10 wt%, most preferably at most 5 wt% of the total liquid composition according to the invention.

According to another embodiment, the liquid nutritional composition further comprising one or more of fat, digestible carbohydrates, and non-digestible carbohydrates.

According to yet another embodiment, said fat provides between 10 to 70 % of the total energy content of the composition, and said digestible carbohydrate provides between 30 to 60 % of the total energy content of the composition. It is customary in the field to recalculate caloric to weight contribution using the Atwater factors for digestible carbohydrates (4 kcal/g), proteins (4 kcal/g) and lipids (9 kcal/g).

According to another embodiment, the protein as comprised by the liquid nutritional composition of the invention provides 5% to 100 %, preferably 10 % to 80 %, more preferably 12 % to 60 %, most preferably 14 % to 30 % of the total energy content of the composition. The high levels of protein are beneficial for patients who may not be physically capable of receiving a large volume, for example, fluid restricted patients. Such patients can be given a reduced level of fluid while still receiving a required amount of nutritional support per day. The composition may be used as a complete nutrition, in addition to or as a replacement for a normal meal consumption. The composition may also be used as a supplement, in addition to normal meal consumption, when the uptake of fats and carbohydrates is of less concern.

The nutritional composition according to the invention is designed to either supplement a person's diet or to provide complete nutritional support. Hence, the composition according to the invention may further comprise at least fat and/or carbohydrate and/or a source of vitamins, minerals, trace elements and/or a source of indigestible carbohydrates. Preferably, the composition according the invention is a nutritionally complete composition.

According to a preferred embodiment, the liquid nutritional composition has a pH of between 6 and 8, comprises between 0.2 and 0.6 g/100 ml lactic acid, and 8 to 12 g of protein per 100 ml of the composition, wherein micellar casein comprises between 60 and 80 wt% and caseinate between 20 and 40 wt% of total protein content, wherein said protein provides 12 to 24 % of the total energy content of the composition, said composition having an energy density of between 1.8 and 2.8 kcal/ml. Said preferred embodiment optionally comprises between 0.015 and 0.1 g/100 ml citric acid and optionally whey in an amount of up to 15wt%, 10 wt%, or up to 5 wt% of total protein content.

According to a yet another preferred embodiment, the liquid nutritional composition has a pH of between 6 and 8, comprises between 0.2 and 0.6 g/100 ml lactic acid, and 12 to 16 g of protein per 100 ml of the composition, wherein micellar casein comprises between 60 and 80 wt% and caseinate between 20 and 40 wt% of total protein content, wherein said protein provides 18 to 30 % of the total energy content of the composition, said composition having an energy density of between 1.8 and 2.8 kcal/ml. This preferred embodiment optionally comprises between 0.015 and 0.1 g/100 ml citric acid and optionally whey in an amount of up to 15 wt%, 10 wt%, or up to 5 wt% of total protein content.

Preferably, the liquid nutritional composition is heat-treated, such as sterilized (e.g. by ultra-high temperature treatment) or pasteurized.

Within the context of the present invention, the terms "heat treatment" and "heat-treated" are meant to comprise any method using heat (preferably sterilization, pasteurization) to reduce the number of or remove possible pathogens. Preferably, a heat treatment includes a heat treatment at a high temperature for a short period, such as a UHT (Ultra High Temperature) treatment.

In one embodiment, the heating conditions are selected in line with those presented in WO-A-03/-11040, its contents herein incorporated by reference. The heat treatment is preferably a temperature of at least 60 °C, preferably at least 70 °C, and less than 200 °C, more preferably less than 160 °C, for a period of time equal to or at least t, which period of heating t is governed by the following formula:

$$t = (500/(T\text{-}59)) - 4,$$

in which t is the duration of heating (in seconds) and T is the heating temperature (in °C). More preferably, the maximum heating conditions complied are governed by the following formula:

$$t = (90000/(T\text{-}59))\text{-}900,$$

in which t and T have the aforesaid meaning. The heat treatment preferably involves a period of 0.1 sec to 24 hour. It is particularly preferred that the heating time ranges from 10 s - 1 hour, more preferably from at least 10 minutes. The preferred corresponding minimum and maximum temperatures may be calculated from the above formulae.

Additionally or alternatively, the "heat treatment" is characterized by a minimum 'sterilizing value' or 'F-zero' (F0) value of at least 2.8 (min), more preferably at least 3 min, most preferably at least 4 minutes, in particular at least 4.5 minutes. It is a standardized and FDA-approved parameter. For any time temperature combination, the sterilizing value F0 is the equivalent minutes at 250 °F. At F0 = 2.8 min, *Clostridium botulinum* is inactivated.

In one embodiment, the preferred heat treatment is sterilization or pasteurization, both having technical meanings well-established in the art. Henceforth, within the context of the present invention, pasteurization is comprised within the term sterilization. Within the context of the present invention, a "heat-treated composition" is a composition that is obtained or obtainable by subjecting a composition to a sterilization treatment. In general, the quantity of potentially pathogenic microorganisms of the sterilized composition meets food safety requirements, as applicable *e.g.* in the US or EU. In particular, a heat-treated composition in accordance with the invention maintains to meet such requirement, for at least 6 months, preferably at least 12 months after packaging, at the beginning of shelf life, when stored in a sealed packaging at ambient temperature (20 °C). It is particularly preferred that changes in stability, e.g. the viscosity, are insignificant over such period, preferably less than 10 % change, more preferably less than 5 % change.

Also encompassed by the present invention is a powder, obtainable by drying the liquid enteral nutritional composition of the present invention.

**Fat as a further component of the composition**

In one embodiment the present enteral nutritional composition further comprises fat. The amount of fat may range between 5 and 95 %, preferably between 10 and 70 %, more preferably between 20 and 40 %, relative to the total energy content of the composition.

With regard to the type of fat, a wide choice is possible, as long as the fat is of food quality. The fat may either be an animal fat or a vegetable fat or both. Although animal fats such as lard or butter have essentially equal caloric and nutritional values and can be used interchangeably, vegetable oils are highly preferred in the practice of the present invention due to their readily availability, ease of formulation, absence of cholesterol and lower concentration of saturated fatty acids. In one embodiment, the present composition comprises rapeseed oil, corn oil and/or sunflower oil.

The fat may include a source of medium chain fatty acids, such as medium chain triglycerides (MCT, mainly 8 to 10 carbon atoms long), a source of long chain fatty acids, such as long chain triglycerides (LCT) and phospholipid-bound fatty acids such as phospholipid-bound EPA or DHA, or any combination of the two types of sources. MCTs are beneficial because they are easily absorbed and metabolized in a metabolically-stressed patient. Moreover, the use of MCTs will reduce the risk of nutrient malabsorption. LCT sources, such as canola oil, rapeseed oil, sunflower oil, soybean oil, olive oil, coconut oil, palm oil, linseed oil, marine oil or corn oil are beneficial because it is known that LCTs may modulate the immune response in the human body.

In one specific embodiment, the fat comprises 30 to 60 weight% of animal, algal or fungal fat, 40 to 70 weight% of vegetable fat and optionally 0 to 20 weight% of MCTs based on total fat of the composition. The animal fat preferably comprises a low amount of milk fat, i.e. lower than 6 weight%, especially lower than 3 weight% based on total fat. In particular, a mixture of corn oil, egg oil, and/or canola oil and specific amounts of marine oil is used. Egg oils, fish oils and algal oils are a preferred source of non-vegetable fats. Especially for compositions that are to be consumed orally, in order to prevent formation of off-flavours and to decrease a fishy after-taste, it is recommended to select ingredients that are relatively low in docosahexaenoic acid (DHA), i.e. less than 6 weight%, preferably less than 4 weight% based on total fat. Marine oils containing DHA are preferably present in the composition according to the invention in an amount lower than 25 weight%, preferably lower than 15 weight% based on total fat. On the other hand, inclusion of eicosapentaenoic acid (EPA) is highly desirable for obtaining the maximum health effect. Therefore, in another embodiment, the amount of EPA may range between 4 weight% and 15 weight%, more preferably between 8 weight% and 13 weight% based on total fat. The weight ratio EPA:DHA is advantageously at least 6:4, for example between 2:1 and 10:1. In yet another embodiment, the amount of EPA is very low, such as 0.1 to 1 weight%, preferably 0.3 weight% or 0.6 weight%, based on total fat.

Also, the nutritional composition according to the invention may beneficially comprise an emulsifier. Commonly known emulsifiers may be used and generally the emulsifier contributes to the energy content of the fat in said composition.

## Digestible carbohydrate as a further component of the composition

In one embodiment of the present invention, the nutritional composition according to the invention further comprises a digestible carbohydrate. Preferably, the digestible carbohydrate provides between 30 to 60 % of the total energy content of the composition according to the invention. The digestible carbohydrate may comprise either simple or complex carbohydrates, or any mixture thereof. Suitable for use in the present invention are glucose, fructose, sucrose, lactose, trehalose, palatinose, corn syrup, malt, maltose, isomaltose, partially hydrolysed corn starch, maltodextrins, glucose oligo- and polysaccharides.

The composition of the digestible carbohydrate preferably is such that high viscosities, excessive sweetness, excessive browning (Maillard reactions) and excessive osmolarities are avoided. Acceptable viscosities and osmolarities may be achieved by adjusting the average chain length (average degree of polymerisation, DP) of the digestible carbohydrates between 1.5 and 6, preferably between 1.8 and 4. In order to avoid excessive sweetness, the total level of sucrose and fructose is preferably less than 60 %, more preferably less than 52 %, more preferably less than 40 % of the weight of the carbohydrate, especially of the digestible carbohydrate. Long-chain digestible carbohydrates such as starch, starch fractions and mild starch hydrolysates (DE > 1, DE < 20), may also be present, preferably in an amount of less than 25 weight%, especially less than 15 weight% of the digestible carbohydrate, and less than 6 g/100 ml, preferably less than 4 g/100 ml of the total enteral composition according to the invention.

In one embodiment of the present invention, the digestible carbohydrate includes maltodextrose with a high DE (dextrose equivalent). In one embodiment the digestible carbohydrate includes maltodextrose with a DE of > 10, preferably a DE of > 20, more preferably > 30 or even > 40, such as a DE of about 47. In one embodiment of the present invention, the digestible carbohydrate includes maltodextrose with a DE >10 and sucrose.

The use of maltodextrose leads to few or no Maillard reaction products upon heating. Without being bound to any explanation, this effect might be attributed to the fact that the compact micellar structure of the micellar casein offers few lysine reaction sites for a Maillard reaction. In one embodiment of the present invention, the digestible carbohydrate includes maltodextrose with a high DE in an amount of at least 35 weight%, preferably at least 50 weight%, preferably at least 65 weight%, preferably at least 90 weight% of the total weight of digestible carbohydrate. In one embodiment of the present invention, the digestible carbohydrate includes maltodextrose with a low DE of 2 to 20. In one embodiment of the present invention, the digestible carbohydrate includes maltodextrose with a low DE of 2 to 10, preferably with a low DE of about 2. In one embodiment of the present invention, the digestible carbohydrate includes maltodextrose with a low DE in an amount of less than 35 weight%, preferably less than 20 weight%, preferably less than 10 weight% of the digestible carbohydrate. Maltodextrose with a low DE may also be referred to as maltodextrine. In another embodiment of the present invention, the digestible carbohydrate includes maltodextrose with a high DE, preferably a DE of > 20, preferably > 30 or even > 40, most preferably a DE of about 47 in combination with maltodextrose with a low DE, preferably a low DE of 2 to 20, more preferably a low DE of 2 to 10, most preferably with a low DE of about 2. As is known, maltodextrose with a low DE, such as of about 2, gives rise to a high viscosity. Maltodextrose with a high DE, such as of about 47 gives rise to a low viscosity, but is very sweet. The combination of both maltodextroses optimizes the balance between sweetness and viscosity. In one embodiment of the present invention, the digestible carbohydrate includes at least 65 weight%, preferably at least 90 weight%, based on total weight of digestible carbohydrate of maltodextrose with a DE >40, preferably with a DE of about 47 and 0 to 10 weight% of maltodextrose with a DE 2 to 10, preferably with a DE of about 2.

In another embodiment of the present invention, the digestible carbohydrate includes trehalose. It is one of the main objects of the invention to provide a nutritional composition with a low viscosity. Sucrose is very well suited for such purpose, but gives rise to very sweet compositions, which are in general disliked by the consumer. Maltodextrose with a low DE, such as of about 2, does not suffer from the latter drawback, but gives rise to a high viscosity. Maltodextrose with a high DE, such as of about 47 gives rise to a low viscosity, but is again very sweet, and gives further rise to the undesired Maillard reactions. Trehalose is a preferred choice of carbohydrate, as it gives rise to a low viscosity, no undesired Maillard reactions and it has a sweetness about half of that of sucrose. In one embodiment of the present invention, the digestible carbohydrate includes trehalose in an amount of 20 % to 60 % of the weight of the carbohydrate, in an amount of 20 % to 45 %, more preferably in an amount of 25 % to 45 % of the weight of the digestible carbohydrate.

**Vitamins, minerals and trace elements as further components of the composition**

The composition according to the invention may contain a variety of vitamins, minerals and trace elements.

In one embodiment of the present invention, the composition according to the invention provides all necessary vitamins, most of the minerals and trace elements. For example, the composition according to the invention preferably provides 6 mg of zinc per 100 ml of the composition which is beneficial for tissue repair in a healing patient. Preferably, the composition according to the invention (also) provides 25 mg of vitamin C per 100 ml of the composition to aid patients with more severe healing requirements. Further, preferably, the composition according to the invention (also) provides 2.25 mg iron per 100 ml of the composition. Iron is beneficial in maintaining bodily fluids as well as circulatory system functions in an elderly patient.

The invention implicates that a composition according to the present invention may contain sodium and/or potassium levels outside FSMP (Foods for Special Medical Purposes) legislation levels.

**Non-digestible carbohydrates as further components of the composition of the invention**

The enteral nutritional composition according to the invention may optionally be fortified with non-digestible carbohydrates (dietary fibres) such as fructo-oligosaccharides or inulin. In an embodiment of the present invention, the composition according to the invention comprises 0.5 g/100 ml to 6 g/100 ml of non-digestible carbohydrates. The dietary fibres include non-digestible oligosaccharides having a DP of 2 to 20, preferably 2 to 10. More preferably, these oligosaccharides do not contain substantial amounts (less than 5 weight%) of saccharides outside these DP ranges, and they are soluble. These oligosaccharides may comprise fructo-oligosaccharides (FOS), trans-galacto-oligosaccharides (TOS), xylo-oligosaccharides (XOS), soy oligosaccharides, and the like. Optionally, also higher molecular weight compounds such as inulin, soy polysaccharides, acacia polysaccharides (acacia fibre or arabic gum), cellulose, resistant starch and the like may be incorporated in the composition according to the invention. The amount of insoluble fibre such as cellulose is preferably lower than 20 weight% of the dietary fibre fraction of the composition according to the invention, and/or below 0.6 g/100 ml. The amount of thickening polysaccharides such as carrageenans, xanthans, pectins, galactomannans and other high molecular weight (DP > 50) indigestible polysaccharides is preferably low, i.e. less than 20 % of the weight of the fibre fraction, or less than 1 g/100 ml. Instead, hydrolysed polysaccharides such as hydrolysed pectins and galactomannans can advantageously be included.

A preferred fibre component is an indigestible oligosaccharide with a chain length (DP) of 2 to 10, for example Fibersol® (resistant oligoglucose), in particular hydrogenated Fibersol®, or a mixture of oligosaccharides having a DP of 2 to 10, such as fructo-oligosaccharides or galacto-oligosaccharides, which may also contain a small amount of higher saccharides (e.g. with a DP of 11 to 20). Such oligosaccharides preferably comprise 50 weight% to 90 weight% of the fibre fraction, or 0.5 g/100 ml to 3 g/100 ml of the composition according to the invention. Other suitable fibre components include saccharides that have only partial digestibility.

In a particular embodiment, the composition according to the invention comprises one or more of fructo-oligosaccharides, inulin, acacia polysaccharides, soy polysaccharides, cellulose and resistant starch.

In another embodiment of the present invention, the composition according to the invention may comprise a mixture of neutral and acid oligosaccharides as disclosed in WO 2005/039597 (N.V. Nutricia), which is incorporated herein by reference in its entirety. More in particular, the acid oligosaccharide has a degree of polymerization (DP) between 1 and 5000, preferably between 1 and 1000, more preferably between 2 and 250, even more preferably between 2 and 50, most preferably between 2 and 10. If a mixture of acid oligosaccharides with different degrees of polymerization is used, the average DP of the acid oligosaccharide mixture is preferably between 2 and 1000, more preferably between 3 and 250, even more preferably between 3 and 50. The acid oligosaccharide may be a homogeneous or heterogeneous carbohydrate. The acid oligosaccharides may be prepared from pectin, pectate, alginate, chondroitine, hyaluronic acids, heparin, heparane, bacterial carbohydrates, sialoglycans, fucoidan, fucooligosaccharides or carrageenan, and are preferably prepared from pectin or alginate. The acid oligosaccharides may be prepared by the methods described in WO 01/60378, which is hereby incorporated by reference. The acid oligosaccharide is preferably prepared from high methoxylated pectin, which is characterized by a degree of methoxylation above 50%. As used herein, "degree of methoxylation" (also referred to as DE or "degree of esterification") is intended to mean the extent to which free carboxylic acid groups contained in the polygalacturonic acid chain have been esterified (e.g. by methylation). The acid oligosaccharides are preferably characterized by a degree of methoxylation above 20%, preferably above 50 % even more preferably above 70%. Preferably the acid oligosaccharides have a degree of methylation above 20%, preferably above 50 % even more preferably above 70%. The acid oligosaccharide is preferably administered in an amount of between 10 mg and 100 gram per day, preferably between 100 mg and 50 grams per day, even more between 0.5 and 20 gram per day.

The term neutral oligosaccharides as used in the present invention refers to saccharides which have a degree of polymerization of monose units exceeding 2, more preferably exceeding 3, even more preferably exceeding 4, most preferably exceeding 10, which are not or only partially digested in the intestine by the action of acids or digestive enzymes present in the human upper digestive tract (small intestine and stomach) but which are fermented by the human intestinal flora and preferably lack acidic groups. The neutral oligosaccharide is structurally (chemically) different from the acid oligosaccharide. The term neutral oligosaccharides as used in the present invention preferably refers to saccharides which have a degree of polymerization of the oligosaccharide below 60 monose units, preferably below 40, even more preferably below 20, most preferably below 10. The term monose units refers to units having a closed ring structure, preferably hexose, e.g. the pyranose or furanose forms. The neutral oligosaccharide preferably comprises at least 90%, more preferably at least 95% monose units selected from the group consisting of mannose, arabinose, fructose, fucose, rhamnose, galactose, beta-D-galactopyranose, ribose, glucose, xylose and derivatives thereof, calculated on the total number of monose units contained therein. Suitable neutral oligosaccharides are preferably fermented by the gut flora. Preferably the oligosaccharide is selected from the group consisting of: cellobiose (4-O-beta-D-glucopyranosyl-D-glucose), cellodextrins ((4-O-beta-D-glucopyranosyl)$_n$-D-glucose), B-cyclodextrins (Cyclic molecules of alpha-1-4-linked D-glucose; alpha-cyclodextrin-hexamer, beta-cyclodextrin-heptamer and gamma-cyclodextrin-octamer), indigestible dextrin , gentiooligosaccharides (mixture of beta-1-6 linked glucose residues, some 1-4 linkages), glucooligosaccharides (mixture of alpha-D-glucose), isomaltooligosaccharides (linear alpha-1-6 linked glucose residues with some 1-4 linkages), isomaltose (6-O-alpha-D-glucopyranosyl-D-glucose); isomaltriose (6-O-alpha-D-glucopyranosyl-(1-6)-alpha-D-glucopyranosyl-D-glucose), panose (6-O-alpha-D-glucopyranosyl-(1-6)-alpha-D-glucopyranosyl-(1-4)-D-glucose), leucrose (5-O-alpha-D-glucopyranosyl-D-fructopyranoside), palatinose or isomaltulose (6-O-alpha-D-glucopyranosyl-D-fructose), theanderose (O-alpha-D-glucopyranosyl-(1-6)-O-alpha-D-glucopyranosyl-(1-2)-B-D-fructofuranoside), D-agatose, D-*lyxo*-hexulose, lactosucrose (O-beta-D-galactopyranosyl-(1-4)-O-alpha-D-glucopyranosyl-(1-2)-beta-D-fructofuranoside), alpha-galactooligosaccharides including raffinose, stachyose and other soy oligosaccharides (O-alpha-D-galactopyranosyl-(1-6)-alpha-D-glucopyranosyl-beta-D-fructofuranoside), beta-galactooligosaccharides or transgalactooligosaccharides (beta-D-galactopyranosyl-(1-6)-[beta-D-glucopyranosyl]$_n$-(1-4) alpha-D glucose), lactulose (4-O-beta-D-galactopyranosyl-D-fructose), 4'-galatosyllactose (O-D-galactopyranosyl-(1-4)-O-beta-D-glucopyranosyl-(1-4)-D-glucopyranose), synthetic galactooligosaccharide (neogalactobiose, isogalactobiose, galsucrose, isolactose I, II and III), fructans - Levan-type (beta-D-(2→6)-fructofuranosyl)$_n$ alpha-D-glucopyranoside), fructans - Inulin-type (beta-D-((2→1)-fructofuranosyl)$_n$ alpha-D-glucopyranoside), 1 f-beta-fructofuranosylnystose (beta-D-((2→1)-fructofuranosyl)$_n$B-D-fructofuranoside), xylooligosaccharides (B-D-((1→4)-xylose)$_n$, lafinose, lactosucrose and arabinooligosaccharides.

According to a further preferred embodiment the neutral oligosaccharide is selected from the group consisting of fructans, fructooligosaccharides, indigestible dextrins galactooligosaccharides (including transgalactooligosaccharides), xylooligosaccharides, arabinooligosaccharides, glucooligosaccharides, mannooligosaccharides, fucooligosaccharides and mixtures thereof Most preferably, the neutral oligosaccharide is selected from the group consisting of fructooligosacchararides, galactooligosaccharides and transgalactooligosaccharides.

Suitable oligosaccharides and their production methods are further described in Laere K.J.M. (Laere, K.J.M., Degradation of structurally different non-digestible oligosaccharides by intestinal bacteria: glycosylhydrolases of Bi. adolescentis. PhD-thesis (2000), Wageningen Agricultural University, Wageningen, The Netherlands), the entire content of which is hereby incorporated by reference. Transgalactooligosaccharides (TOS) are for example sold under the trademark Vivinal™ (Borculo Domo Ingredients, Netherlands). Indigestible dextrin, which may be produced by pyrolysis of corn starch, comprises alpha(1→4) and alpha(1→6) glucosidic bonds, as are present in the native starch, and contains 1→2 and 1→3 linkages and levoglucosan. Due to these structural characteristics, indigestible dextrin contains well-developed, branched particles that are partially hydrolysed by human digestive enzymes. Numerous other commercial sources of indigestible oligosaccharides are readily available and known to skilled person. For example, transgalactooligosaccharide is available from Yakult Honsha Co., Tokyo, Japan. Soybean oligosaccharide is available from Calpis Corporation distributed by Ajinomoto U.S.A. Inc., Teaneck, N.J.

In a further preferred embodiment, the composition according to the invention comprises an acid oligosaccharide with a DP between 2 and 250, prepared from pectin, alginate, and mixtures thereof; and a neutral oligosaccharide, selected from the group of fructans, fructooligosaccharides, indigestible dextrins, galactooligosaccharides including transgalactooligosaccharides, xylooligosaccharides, arabinooligosaccharides, glucooligosaccharides, mannooligosaccharides, fucooligosaccharides, and mixtures thereof.

In a further preferred embodiment the composition according to the invention comprises two chemically distinct neutral oligosaccharides. It was found that the administration of acid oligosaccharides combined with two chemically distinct neutral oligosaccharides provides an optimal synergistic immune stimulatory effect. Preferably the composition according to the invention comprises:

an acid oligosaccharides as defined above ;

a galactose-based neutral oligosaccharide (of which more than 50 % of the monose units are galactose units), preferably selected from the group consisting of galactooligosaccharide and transgalactooligosaccharide; and

a fructose and/or glucose based neutral oligosaccharide (of which more than 50% of the monose units are fructose and/or glucose, preferably fructose units), preferably inulin, fructan and/or fructooligosaccharide, most preferably long chain fructooligosaccharide (with an average DP of 10 to 60).

The mixture of acid- and neutral oligosaccharides is preferably administered in an amount of between 10 mg and 100 gram per day, preferably between 100 mg and 25 grams per day, even more preferably between 0.5 and 20 gram per day.

## Dosage unit and the nutritional compositions for use in treatment

Further encompassed by the present invention is the nutritional composition of the present invention for use in treating an elderly person of the age of 50 or more, a person that is in a disease state, a person that is recovering from a disease state, or a person that is malnourished.

The energy density of the liquid nutritional composition of the present invention preferably lies between 1.2 and 3.5 kcal/ml, more preferably between 1.4 and 3.0 kcal/ml, most preferably between 1.8 and 2.8 kcal/ml.

The enteral nutritional composition according to the invention may have the form of a complete food, *i.e.* it can meet all nutritional needs of the user. As such, it preferably contains 1200 to 2500 kcal per daily dosage. The daily dosage amounts are given with respect to a daily energy supply of 2000 kcal to a healthy adult having a body weight of 70 kg. For persons of different condition and different body weight, the levels should be adapted accordingly. It is understood that the average daily energy intake preferably is about 2000 kcal. The complete food can be in the form of multiple dosage units, e.g. from 4 (250 ml/unit) to 40 (20 ml/unit) per day for an energy supply of 2000 kcal/day using an enteral nutritional composition of 2.0 kcal/ml.

The enteral nutritional composition can also be a food supplement, for example to be used in addition to a non-medical food. Preferably as a supplement, the enteral nutritional composition contains per daily dosage less than 1500 kcal, in particular as a supplement, the enteral nutritional composition contains 400 to 1000 kcal per daily dose. The food supplement can be in the form of multiple dosage units, e.g. from 2 (250 ml/unit) to 10 (50 ml/unit) per day for an energy supply of 1000 kcal/day using a enteral nutritional composition of 2.0 kcal/ml.

In one embodiment of the present invention, a unit dosage comprises any amount of the enteral nutritional composition according to the invention between 10 ml and 250 ml, the end values of this range included, preferably any amount between 25 ml and 225 ml, the end values of this range included, more preferably any amount between 100 ml and 200 ml, the end values of this range included, most preferably about 125 ml or about 200ml. For example, a person receiving 50 ml unit dosages can be given 10 unit dosages per day to provide nutritional support using a enteral nutritional composition of 2.0 kcal/ml. Alternatively a person receiving 125 ml unit dosages can be given 4 or 5 or 6 or 7 or 8 unit dosages per day to provide nutritional support using a enteral nutritional composition of 2.0 kcal/ml. Such small dosage units are preferred because of better compliance.

In one embodiment of the present invention, the composition is provided in a ready to use form and does not require reconstitution or mixing prior to use. The composition according to the invention can be tube fed or administered orally. For example, the composition according to the invention can be provided in a can, on spike, and hang bag. However, a composition may be provided to a person in need thereof in powder form, suitable for reconstitution using an aqueous solution or water such that the composition according to the invention is produced. Thus in one embodiment of the present invention, the present composition is in the form of a powder, accompanied with instructions to dissolve or reconstitute in an aqueous composition or water to arrive at the nutritional enteral composition according to the present invention. In one embodiment of the present invention, the present nutritional enteral composition may thus be obtained by dissolving or reconstituting a powder, preferably in an aqueous composition, in particular water.

In one embodiment of the present invention, the composition according to the invention is packaged. The packaging may have any suitable form, for example a block-shaped carton, e.g. to be emptied with a straw; a carton or plastic beaker with removable cover ; a small-sized bottle for example for the 80 ml to 200 ml range, and small cups for example for the 10 ml to 30 ml range. Another suitable packaging mode is inclusion of small volumes of (e.g. 10 ml to 20 ml) in edible solid or semi-solid hulls or capsules, for example gelatine-like coverings and the like. Another suitable packaging mode is a powder in a container, e.g. a sachet, preferably with instructions to dissolve or reconstitute in an aqueous composition or water.

Thus, further encompassed by the present invention is the nutritional composition of the present invention for use in treating an elderly person of the age of 50 or more, a person that is in a disease state, a person that is recovering from a disease state, or a person that is malnourished. In this respect, it is submitted that in the context of this application, an elderly person is a person of the age of 50 or more, in particular of the age of 55 or more, more in particular of the age of 60 or more, more in particular of the age of 65 or more.

## Viscosity and osmolarity of the nutritional composition

In the context of this invention, viscosity is measured at a shear rate of 100 s$^{-1}$ at 20 °C using a rotational viscosity meter using a cone/plate geometry.

In one embodiment, the viscosity of the liquid nutritional composition of the present invention is less than 200 mPa.s, more preferably less than 150 mPa.s, even more preferably less than 100 mPa.s, most preferably less than 80 mPa.s.

In a preferred embodiment, the viscosity of the liquid nutritional composition of the present invention comprising lactic acid in a selected weight amount is less than if the same composition comprises citric acid in the same selected weight amount. A low viscosity is ideal for orally administering the liquid enteral nutritional composition according to the invention because a person may easily consume a serving having a low viscosity such as that displayed by the present invention. This is also ideal for unit dosages that are tube fed.

In one embodiment of the present invention, the osmolarity of the composition is preferably lower than 1200 mOsm/l, more preferably lower than 1000 mOsm/l, most preferably lower than 900 mOsm/l.

## Method of reducing viscosity

Also encompassed by the present invention is a method of reducing viscosity of a liquid nutritional composition having a pH in a range of 6 to 8, comprising 6 to 20 g/100 ml protein of which at least 55 wt% is micellar casein, comprising the step of including lactic acid in said composition, wherein the lactic acid is present in an amount between 0.05 and up to 1.5 g/100 ml. This is preferably achieved by including lactic acid instead of citric acid as an organic acid.

The liquid nutritional composition according to the method of reducing viscosity preferably comprises an amount of protein which lies between 7 and 20 g/100 ml, preferably between 8 and 18 g/100 ml, more preferably between 9 and 16 g/100 ml.

The liquid nutritional composition according to the method of reducing viscosity preferably comprises an amount of micellar casein of at least 60 wt% based on total protein content, preferably lies between 60 and 95 wt%, more preferably between 65 and 90 wt%, most preferably between 65 and 80 wt% based on total protein content.

The liquid nutritional composition according to the method of reducing viscosity preferably further comprises caseinate, preferably at most 40 wt%, more preferably at most 35 wt%, based on total protein content.

The liquid nutritional composition according to the method of reducing viscosity preferably comprises lactic acid in an amount between 0.05 and up to 1.5 g/100ml, preferably the amount of lactic acid lies between 0.05 and 1.0 g/100 ml, more preferably between 0.1 and 1.0 g/100ml, most preferably between 0.2 and 0.5 g/100ml of the total liquid composition.

The liquid nutritional composition according to the method of reducing viscosity preferably comprises an amount of lactic acid which up to 400 mg/g micellar casein of the total liquid composition, preferably the amount lies between 4 and 300 mg/g protein, more preferably between 10 and 200 mg/g micellar casein, most preferably between 20 and 100 mg lactic acid per g micellar casein of the total liquid composition.

The liquid nutritional composition according to the method of reducing viscosity preferably further comprises citrate, preferably in an amount up to 1 g/100ml, preferably in an amount between 1 and 500 mg/100ml, more preferably in an amount between 5 and 400 mg/100ml, more preferably in an amount between 10 and 300 mg/100ml, most preferably in an amount of 15 and 100 mg/100ml of the total liquid composition.

In the liquid nutritional composition according to the method of reducing viscosity, the weight amount of lactic acid preferably exceeds the weight amount of citrate, preferably by a factor 1.1 to 20, more preferably by a factor 2 to 18, more preferably a factor 3 to 15 or 4 to 12.

The liquid nutritional composition according to the method of reducing viscosity is preferably heat-treated as meant herein.

The liquid nutritional composition according to the method of reducing viscosity preferably has a viscosity which is lower than 200 mPa.s, more preferably lower than 150 mPa.s, even more preferably lower than 100 mPa.s, or most preferably lower than 80 mPa.s, as measured at a shear rate of 100 s$^{-1}$ at 20 °C using a rotational viscosity meter using a cone/plate geometry.

## Use of lactic acid in reducing viscosity of the nutritional compositions

Also encompassed by the present invention is the use of lactic acid for reducing viscosity of a liquid nutritional composition, said protein comprising micellar casein, wherein the amount of micellar casein is at least 55 wt% based on total protein content, wherein the lactic acid is present in an amount between 0.05 and up to 1.5 g/100 ml. Said composition has a pH in a range of 6 to 8 and comprises 6 to 20 g/100 ml protein, said protein comprising micellar casein.

## FIGURES

Figure 1: Effect of addition of lactate or citrate on viscosity of a liquid high-protein nutritional composition comprising micellar casein. X-axis: concentration of citrate or lactate (g/100ml), Y-axis: viscosity (measured at shear rate 100 s-1 mPa.s, at 20°C). Open squares: Citrate concentration (g/100ml), closed triangles: Lactate concentration (g/100ml).

The invention will now be further elucidated by several examples, without being limited thereby.

## EXPERIMENTAL

**Comparative example: effects of lactic acid and citric acid on viscosity**

*Materials and methods*

Proteins (sodium caseinate and micellar casein isolate) and any carbohydrates were dissolved in tap water to obtain a master batch. The temperature of the water was around 60°C to facilitate dissolving of proteins in the water phase. Pre-solutions of minerals were made and subsequently added and stirred through the master batch. These pre-solutions contained varying amounts of citrate or lactate. At this stage, the pH of the master batch was between pH 6.4 and 6.8. The master batch was subsequently de-aerated for approximately 1 hour before emulsification was started. When included, any oils and/or fats were at this stage added to the master batch after which standard homogenization and pasteurization steps were performed. Thereafter, any flavours, colouring agents, and vitamins were stirred through the master batch and the pH was adjusted to a pH value between 6.5 and 6.8. Finally, tap water was added to the master batch to obtain a liquid nutritional composition with 9.6 g/100 ml protein, of which 70 wt% was micellar casein isolate and 30 wt% sodium caseinate. Sterilization was performed at 124°C for 4 min. All components were commercially available.

The levels of potassium lactate and tri-potassium citrate or tri-sodium citrate and citric acid were varied in order to investigate viscosity levels, as indicated in Figure 1. Throughout the comparison, variations in other components, such as sodium and potassium, were kept at a minimum.

*Results*

Inclusion of increasing amounts of citrate (*i.e.* tri-sodium citrate, tri-potassium citrate and citric acid as citrate sources) led to a marked increase in viscosity, whereas the increase in viscosity was significantly less when the same levels of lactate (*i.e.* potassium lactate as lactate source) were included in the composition. Using lactate instead of citrate thus allows one to achieve a reduction in viscosity of compositions that are further identical, equal or at least comparable (e.g. with respect to protein content). Furthermore, it allows one to modulate other relevant parameters, such as providing increased energy density or decreased water content and the like, while viscosity remains at the same level as if citrate was used. A further consequence of this finding is that lactate as an anion can be used as a vehicle to allow the inclusion of higher levels of metal ions, such as potassium but also sodium, without the increased viscosity associated therewith if citrate would have been used. This can be of relevance in the development of foods for special medical purposes that require considerable amounts as minimum levels of a variety of ingredients, such as metal ions.

The demonstrated viscosity behaviour of lactic acid in relation to citric acid was also observed under different circumstances or with different compositions also falling within the scope of the invention, such as when production of liquid nutritional compositions was scaled up. Again, under such conditions, viscosity of the compositions comprising only lactate stabilized (*i.e.* stayed below 100 or 80 mPa.s), whereas viscosity of comparable compositions with citrate increased more significantly. The viscosities plotted in Figure 1 should thus merely be regarded as indicative: In all events, lactic acid guaranteed acceptable liquid formulations, but citric acid did not. Similarly, when combinations of lactate and citrate where used, viscosity levels stabilized and did not increase as much as when only citrate was used in similar amounts.

## Claims

1. A liquid nutritional composition having a pH in a range of 6 to 8, comprising 6 to 20 g/100 ml protein, said protein comprising micellar casein, wherein the amount of micellar casein is at least 55 wt% based on total protein content, and wherein lactic acid is present in an amount between 0.05 and up to 1.5 g/100 ml.

2. The liquid nutritional composition of claim 1, wherein the amount of protein lies between 7 and 20 g/100 ml, preferably between 8 and 18 g/100 ml, more preferably between 9 and 16 g/100 ml.

3. The liquid nutritional composition of claim 1 or 2, wherein the amount of micellar casein lies between 55 and 95 wt%, more preferably between 60 and 90 wt%, most preferably between 65 and 85 wt% based on total protein content, and said liquid nutritional composition preferably comprising caseinate, preferably at most 40 wt%, more preferably at most 35 wt%, based on total protein content.

4. The liquid nutritional composition according to any of the preceding claims, wherein the amount of lactic acid lies between 0.05 and 1.0 g/100 ml, more preferably between 0.1 and 1.0 g/100ml, most preferably between 0.2 and 0.5 g/100ml, most preferably at least 0.3 g/100 ml, in particular at least 0.35 g/100 ml of the total liquid composition.

5. The liquid nutritional composition according to any of the preceding claims, wherein the amount of lactic acid is up to 250 mg/g protein, preferably the amount of lactic acid lies between 1 and 200 mg per gram protein of the total liquid composition, preferably between 2.5 and 100 mg/g protein, more preferably between 5 and 75 mg/g protein, most preferably between 10 and 75 mg lactic acid, per gram protein of the total liquid composition; and/or wherein the amount of lactic acid is up to 400 mg/g micellar casein of the total liquid composition, preferably the amount lies between 4 and 300 mg/g protein, more preferably between 10 and 200 mg/g micellar casein, most preferably between 20 and 100 mg lactic acid per g micellar casein of the total liquid composition

6. The liquid nutritional composition according to any of the preceding claims, wherein the composition further comprises citrate, preferably in an amount up to 1 g/100ml, preferably in an amount between 1 and 500 mg/100ml, more preferably in an amount between 5 and 400 mg/100ml, more preferably in an amount between10 and 300 mg/100ml, most preferably in an amount of 15 and 100 mg/100ml of the total liquid composition; and/or wherein the weight amount of lactic acid exceeds the weight amount of citrate, preferably by a factor 1.1 to 20, more preferably by a factor 2 to 18, more preferably a factor 3 to 15 or most preferably 4 to 12.

7. The liquid nutritional composition according to any of the preceding claims, wherein the composition has an energy density of between 1.2 and 3.5 kcal/ml, preferably between 1.4 and 3.0 kcal/ml, more preferably between 1.8 and 2.8 kcal/ml.

8. The liquid nutritional composition according to any of the preceding claims, further comprising at least one monovalent metal ion, wherein the total amount of monovalent metal ions comprised by the liquid composition preferably lies between 50 and 700 mg/100ml, preferably between 100 and 600, more preferably between 125 and 500, most preferably between 125 and 400; and/or wherein the total amount of monovalent metal ions comprised by the liquid composition lies preferably between 25 and 400 mg/100 kcal, preferably between 30 and 300, more preferably between 40 and 350, most preferably between 50 and 300 mg/100 kcal.

9. The liquid nutritional composition according to any of the preceding claims, wherein the composition further comprises at least one divalent metal ion, preferably calcium and/or magnesium, wherein the total amount of divalent metal ions is preferably present in an amount of between 10 and 600 mg/100ml, preferably between 50 and 550 mg/100 ml, more preferably between 100 and 500 mg/100ml; and/or wherein the total amount of divalent metal ions is present in an amount of between 30 and 400 mg/100kcal, preferably between 50 and 350 mg/100 kcal, more preferably between 60 and 300 mg/100 kcal.

10. The liquid nutritional composition according to any of the preceding claims, wherein the nutritional composition is obtained or obtainable by subjecting said liquid nutritional composition having a pH in a range of 6 to 8, comprising 6 to 20 g/100 ml protein, said protein comprising micellar casein, and wherein lactic acid is present in an amount between 0.05 and up to 1.5 g/100 ml to a heat-treatment, preferably a pasteurization or sterilization treatment.

11. The liquid nutritional composition according to any of the preceding claims, wherein the viscosity of the composition is lower than 200 mPa.s, preferably lower than 150 mPa.s, more preferably lower than 100 mPa.s, or lower than 80 mPa.s, as measured at a shear rate of 100 s-1 at 20 °C using a rotational viscosity meter using a cone/plate geometry.

12. A powder, obtainable from the liquid enteral nutritional composition according to any one of claims 1 to 11.

13. Liquid nutritional composition according to any of claims 1 - 11, or powder according to claim 12 for use in treating a person of the age of 50 or more, a person that is in a disease state, a person that is recovering from a disease state, or a person that is malnourished.

14. A method of reducing viscosity of a liquid nutritional composition having a pH in a range of 6 to 8, comprising 6 to 20 g/100 ml protein of which at least 55 wt% is micellar casein, comprising the step of including lactic acid in said composition, wherein the lactic acid is present in an amount between 0.05 and up to 1.5 g/100 ml.

15. A liquid nutritional composition obtainable by the method according to claim 14.

16. Use of lactic acid for reducing viscosity of a liquid nutritional composition, said composition having a pH in a range of 6 to 8 and comprising 6 to 20 g/100 ml protein, said protein comprising micellar casein, wherein the amount of micellar casein is at least 55 wt% based on total protein content, wherein the lactic acid is present in an amount between 0.05 and up to 1.5 g/100 ml.

**Patentansprüche**

1. Eine flüssige Ernährungszusammensetzung mit einem pH-Wert im Bereich von 6 bis 8, umfassend 6 bis 20 g/100 ml Protein, wobei das Protein mizellares Casein umfasst, wobei die Menge an mizellarem Casein mindestens 55 Gew.-% bezogen auf den gesamten Proteingehalt beträgt, und wobei Milchsäure in einer Menge zwischen 0,05 und bis zu 1,5 g/100 ml vorhanden ist.

2. Die flüssige Ernährungszusammensetzung nach Anspruch 1, wobei die Menge an Protein zwischen 7 und 20 g/100 ml liegt, vorzugsweise zwischen 8 und 18 g/100 ml, weiter bevorzugt zwischen 9 und 16 g/100 ml.

3. Die flüssige Ernährungszusammensetzung nach Anspruch 1 oder 2, wobei die Menge an mizellarem Casein zwischen 55 und 95 Gew.-% liegt, weiter bevorzugt zwischen 60 und 90 Gew.-%, am meisten bevorzugt zwischen 65 und 85 Gew.-%, bezogen auf den gesamten Proteingehalt, und wobei die flüssige Ernährungszusammensetzung vorzugsweise Caseinat umfasst, vorzugsweise höchstens 40 Gew.-%, weiter bevorzugt höchstens 35 Gew.-%, bezogen auf den gesamten Proteingehalt.

4. Die flüssige Ernährungszusammensetzung nach einem der vorhergehenden Ansprüche, wobei die Menge an Milchsäure zwischen 0,05 und 1,0 g/100 ml liegt, weiter bevorzugt zwischen 0,1 und 1,0 g/100 ml, am meisten bevorzugt zwischen 0,2 und 0,5 g/100 ml, am meisten bevorzugt mindestens 0,3 g/100 ml, insbesondere mindestens 0,35 g/100 ml, bezogen auf die gesamte flüssige Zusammensetzung.

5. Die flüssige Ernährungszusammensetzung nach einem der vorhergehenden Ansprüche, wobei die Menge an Milchsäure bis zu 250 mg/g Protein beträgt, vorzugsweise liegt die Menge an Milchsäure zwischen 1 und 200 mg pro Gramm Protein der gesamten flüssigen Zusammensetzung, vorzugsweise zwischen 2,5 und 100 mg/g Protein, weiter bevorzugt zwischen 5 und 75 mg/g Protein, am meisten bevorzugt zwischen 10 und 75 mg Milchsäure, pro Gramm Protein der gesamten flüssigen Zusammensetzung; und/oder wobei die Menge an Milchsäure bis zu 400 mg/g mizellares Casein der gesamten flüssigen Zusammensetzung beträgt, vorzugsweise liegt die Menge zwischen 4 und 300 mg/g Protein, weiter bevorzugt zwischen 10 und 200 mg/g mizellares Casein, am meisten bevorzugt zwischen 20 und 100 mg Milchsäure pro g mizellares Casein der gesamten flüssigen Zusammensetzung.

6. Die flüssige Ernährungszusammensetzung nach einem der vorhergehenden Ansprüche, wobei die Zusammensetzung ferner Citrat umfasst, vorzugsweise in einer Menge von bis zu 1 g/100 ml, vorzugsweise in einer Menge zwischen 1 und 500 mg/100 ml, weiter bevorzugt in einer Menge zwischen 5 und 400 mg/100 ml, weiter bevorzugt in einer Menge zwischen 10 und 300 mg/100 ml, am meisten bevorzugt in einer Menge von 15 und 100 mg/100 ml, der gesamten flüssigen Zusammensetzung; und/oder wobei die Gewichtsmenge an Milchsäure die Gewichtsmenge an Citrat übersteigt, vorzugsweise um einen Faktor 1,1 bis 20, weiter bevorzugt um einen Faktor 2 bis 18, weiter bevorzugt einen Faktor 3 bis 15 oder am meisten bevorzugt 4 bis 12.

7. Die flüssige Ernährungszusammensetzung nach einem der vorhergehenden Ansprüche, wobei die Zusammensetzung eine Energiedichte zwischen 1,2 und 3,5 kcal/ml aufweist, vorzugsweise zwischen 1,4 und 3,0 kcal/ml, weiter bevorzugt zwischen 1,8 und 2,8 kcal/ml.

8. Die flüssige Ernährungszusammensetzung nach einem der vorhergehenden Ansprüche, die ferner mindestens ein monovalentes Metallion umfasst, wobei die gesamte Menge an einwertigen Metallionen, die in der flüssigen Zusammensetzung enthalten ist, vorzugsweise zwischen 50 und 700 mg/100 ml liegt, vorzugsweise zwischen 100 und 600, weiter bevorzugt zwischen 125 und 500, am meisten bevorzugt zwischen 125 und 400; und/oder wobei die gesamte Menge an monovalenten Metallionen, die in der flüssigen Zusammensetzung enthalten sind, vorzugsweise zwischen 25 und 400 mg/100 kcal liegt, vorzugsweise zwischen 30 und 300, weiter bevorzugt zwischen 40 und 350, am meisten bevorzugt zwischen 50 und 300 mg/100 kcal.

9. Die flüssige Ernährungszusammensetzung nach einem der vorhergehenden Ansprüche, wobei die Zusammensetzung ferner mindestens ein zweiwertiges Metallion umfasst, vorzugsweise Calcium und/oder Magnesium, wobei die gesamte Menge an zweiwertigen Metallionen vorzugsweise in einer Menge zwischen 10 und 600 mg/100 ml vorhanden ist, vorzugsweise zwischen 50 und 550 mg/100 ml, weiter bevorzugt zwischen 100 und 500 mg/100 ml; und/oder wobei die gesamte Menge an zweiwertigen Metallionen in einer Menge zwischen 30 und 400 mg/100 kcal vorhanden ist, vorzugsweise zwischen 50 und 350 mg/100 kcal, weiter bevorzugt zwischen 60 und 300 mg/100 kcal.

10. Die flüssige Ernährungszusammensetzung nach einem der vorhergehenden Ansprüche, wobei die Ernährungszusammensetzung erhalten oder erhältlich ist, indem die flüssige Ernährungszusammensetzung, die einen pH-Wert in einem Bereich von 6 bis 8 aufweist, 6 bis 20 g/100 ml Protein umfasst, wobei das Protein mizellares Casein umfasst, und wobei Milchsäure in einer Menge zwischen 0,05 und bis zu 1,5 g/100 ml vorhanden ist, einer Wärmebehandlung unterworfen wird, vorzugsweise einer Pasteurisierungs- oder Sterilisationsbehandlung.

**11.** Die flüssige Ernährungszusammensetzung nach einem der vorhergehenden Ansprüche, wobei die Viskosität der Zusammensetzung weniger als 200 mPa·s beträgt, vorzugsweise weniger als 150 mPa·s, weiter bevorzugt weniger als 100 mPa·s oder weniger als 80 mPa·s, wie bei einer Schergeschwindigkeit von 100 s-1 bei 20 °C unter Verwendung eines Rotationsviskositätsmessers unter Verwendung einer Kegel/Plattengeometrie gemessen.

**12.** Ein Pulver, erhältlich aus der flüssigen enteralen Ernährungszusammensetzung nach einem der Ansprüche 1 bis 11.

**13.** Flüssige Ernährungszusammensetzung nach einem der Ansprüche 1-11 oder Pulver nach Anspruch 12 zur Verwendung bei der Behandlung einer Person mit einem Alter von 50 oder höher, einer Person, die in einem Krankheitszustand ist, einer Person, die sich von einem Krankheitszustand erholt, oder einer Person, die unterernährt ist.

**14.** Ein Verfahren zur Verminderung der Viskosität einer flüssigen Ernährungszusammensetzung mit einem pH-Wert im Bereich von 6 bis 8, die 6 bis 20 g/100 ml Protein umfasst, von dem mindestens 55 Gew.-% mizellares Casein sind, umfassend den Schritt des Einbeziehens von Milchsäure in die Zusammensetzung, wobei die Milchsäure in einer Menge zwischen 0,05 und bis zu 1,5 g/100 ml vorhanden ist.

**15.** Eine flüssige Ernährungszusammensetzung, erhältlich durch das Verfahren nach Anspruch 14.

**16.** Verwendung von Milchsäure zur Verminderung der Viskosität einer flüssigen Ernährungszusammensetzung, wobei die Zusammensetzung einen pH-Wert im Bereich von 6 bis 8 aufweist und 6 bis 20 g/100 ml Protein umfasst, wobei das Protein mizellares Casein umfasst, wobei die Menge an mizellarem Casein mindestens 55 Gew.-% bezogen auf den gesamten Proteingehalt beträgt, wobei die Milchsäure in einer Menge zwischen 0,05 und bis zu 1,5 g/100 ml vorhanden ist.

**Revendications**

**1.** Composition nutritionnelle liquide ayant un pH dans une plage de 6 à 8, comprenant 6 à 20 g/100 ml de protéine, ladite protéine comprenant de la caséine micellaire, dans laquelle la quantité de caséine micellaire est d'au moins 55 % en poids sur la base de la teneur en protéine totale, et dans laquelle l'acide lactique est présent en une quantité comprise entre 0,05 et jusqu'à 1,5 g/100 ml.

**2.** Composition nutritionnelle liquide selon la revendication 1, dans laquelle la quantité de protéine est comprise entre 7 et 20 g/100 ml, de préférence entre 8 et 18 g/100 ml, de manière plus préférée entre 9 et 16 g/100 ml.

**3.** Composition nutritionnelle liquide selon la revendication 1 ou 2, dans laquelle la quantité de caséine micellaire est comprise entre 55 et 95 % en poids, de préférence entre 60 et 90 % en poids, de manière encore plus préférée entre 65 et 85 % en poids sur la base de la teneur en protéine totale, et ladite composition nutritionnelle liquide comprend de préférence du caséinate, de préférence à plus de 40 % en poids, de manière plus préférée à plus de 35 % en poids sur la base de la teneur en protéine totale.

**4.** Composition nutritionnelle liquide selon l'une quelconque des revendications précédentes, dans laquelle la quantité d'acide lactique est comprise entre 0,05 et 1,0 g/100 ml, de préférence entre 0,1 et 1,0 g/100 ml, de manière plus préférée entre 0,2 et 0,5 g/100 ml, de manière encore plus préférée au moins 0,3 g/100 ml, en particulier au moins 0,35 g/100 ml de la composition liquide totale.

**5.** Composition nutritionnelle liquide selon l'une quelconque des revendications précédentes, dans laquelle la quantité d'acide lactique est inférieure ou égale à 250 mg/g de protéine, de préférence la quantité d'acide lactique est comprise entre 1 et 200 mg par gramme de protéine de la composition liquide totale, de préférence entre 2,5 et 100 mg/g de protéine, de manière plus préférée entre 5 et 75 mg/g de protéine, de manière encore plus préférée entre 10 et 75 mg d'acide lactique, par gramme de protéine de la composition liquide totale ; et/ou dans laquelle la quantité d'acide lactique est inférieure ou égale à 400 mg/g de caséine micellaire de la composition liquide totale, de préférence la quantité est comprise entre 4 et 300 mg/g de protéine, de manière plus préférée entre 10 et 200 mg/g de caséine micellaire, de manière encore plus préférée entre 20 et 100 mg d'acide lactique par gramme de caséine micellaire de la composition liquide totale.

**6.** Composition nutritionnelle liquide selon l'une quelconque des revendications précédentes, dans laquelle la composition comprend en outre du citrate, de préférence en une quantité inférieure ou égale à 1 g/100 ml, de préférence en une quantité entre 1 et 500 mg/100 ml, de manière plus préférée en une quantité entre 5 et 400 mg/100 ml, de manière plus préférée en une quantité entre 10 et 300 mg/100 ml, de manière encore plus préférée en une quantité entre 15 et 100 mg/100 ml de la composition liquide totale ; et/ou dans laquelle la quantité en poids d'acide lactique excède la quantité en poids de citrate, de préférence par un facteur de 1,1 à 20, de manière plus préférée par un facteur de 2 à 18, de manières plus préférée un facteur de 3 à 15, ou de manière encore plus préférée de 4 à 12.

7. Composition nutritionnelle liquide selon l'une quelconque des revendications précédentes, dans laquelle la composition a une densité d'énergie entre 1,2 et 3,5 kcal/ml, de préférence entre 1,4 et 3,0 kcal/ml, de manière plus préférée entre 1,8 et 2,8 kcal/ml.

8. Composition nutritionnelle liquide selon l'une quelconque des revendications précédentes comprenant en outre au moins un ion métallique monovalent, dans laquelle la quantité totale d'ions métalliques monovalents contenue dans la composition liquide est de préférence comprise entre 50 et 700 mg/100 ml, de préférence entre 100 et 600, de manière plus préférée entre 125 et 500, de manière encore plus préférée entre 125 et 400 ; et/ou dans laquelle la quantité totale d'ions métalliques monovalents contenue dans la composition liquide est de préférence comprise entre 25 et 400 mg/100 kcal, de préférence entre 30 et 300, de manière plus préférée entre 40 et 350, de manière encore plus préférée entre 50 et 300 mg/100 kcal.

9. Composition nutritionnelle liquide selon l'une quelconque des revendications précédentes, dans laquelle la composition comprend en outre au moins un ion métallique divalent, de préférence calcium et/ou magnésium, dans laquelle la quantité totale d'ions métalliques divalents est de préférence présente en une quantité entre 10 et 600 mg/100 ml, de préférence entre 50 et 550 mg/100 ml, de manière plus préférée entre 100 et 500 mg/100 ml ; et/ou dans laquelle la quantité totale d'ions métalliques divalents est présente en une quantité comprise entre 30 et 400 mg/100 kcal, de préférence entre 50 et 350 mg/100 kcal, de manière plus préférée entre 60 et 300 mg/100 kcal.

10. Composition nutritionnelle liquide selon l'une quelconque des revendications précédentes, dans laquelle la composition nutritionnelle est obtenue ou peut être obtenue en soumettant ladite composition nutritionnelle liquide ayant un pH dans une plage allant de 6 à 8, comprenant entre 6 à 20 g/100 ml de protéine, ladite protéine comprenant de la caséine micellaire, et dans laquelle l'acide lactique est présent en une quantité entre 0,05 et jusqu'à 1,5 g/100 ml à un traitement thermique, de préférence un traitement de pasteurisation ou stérilisation.

11. Composition nutritionnelle liquide selon l'une quelconque des revendications précédentes, dans laquelle la viscosité de la composition est inférieure à 200 mPa.s, de préférence inférieure à 150 mPa.s, de manière plus préférée inférieure à 100 mPa.s, ou inférieure à 80 mPa.s, lorsque mesurée à un taux de cisaillement de 100 s-1 à 20°C en utilisant un viscosimètre rotatif utilisant une géométrie de cône/plaque.

12. Poudre, pouvant être obtenue à partir de la composition nutritionnelle entérale liquide selon l'une quelconque des revendications 1 à 11.

13. Composition nutritionnelle liquide selon l'une quelconque des revendications 1-11, ou poudre selon la revendication 12 à utiliser dans le traitement d'une personne âgée de 50 ans ou plus, une personne qui est dans un état maladif, une personne qui récupère d'un état maladif, ou une personne qui est malnutrie.

14. Procédé pour réduire la viscosité d'une composition nutritionnelle liquide ayant un pH dans une plage allant de 6 à 8, comprenant 6 à 20 g/100 ml de protéine dont au moins 55 % en poids sont de la caséine micellaire, comprenant l'étape consistant à inclure de l'acide lactique dans ladite composition, dans lequel l'acide lactique est présent une quantité entre 0,05 et jusqu'à 1,5 g/100 ml.

15. Composition nutritionnelle liquide pouvant être obtenue par le procédé selon la revendication 14.

16. Utilisation d'acide lactique pour réduire la viscosité d'une composition nutritionnelle liquide, ladite composition ayant un pH dans une plage allant de 6 à 8 et comprenant 6 à 20 g/100 ml de protéine, ladite protéine comprenant de la caséine micellaire, dans laquelle la quantité de caséine micellaire est au moins de 55 % en poids sur la base de la teneur en protéine totale, dans laquelle l'acide lactique est présent en une quantité entre 0,05 et jusqu'à 1,5 g/100 ml.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200230210 A
- WO 2004054371 A
- WO 2010140877 A
- WO 0311040 A
- WO 2005039597 A
- WO 0160378 A

### Non-patent literature cited in the description

- **WALSTRA et al.** Dairy Science and Technology. CRC Press, 2006, 4
- Degradation of structurally different non-digestible oligosaccharides by intestinal bacteria: glycosylhydrolases of Bi. adolescentis. **LAERE, K.J.M.** PhD-thesis. Wageningen Agricultural University, 2000